# EUROPÄISCHE PATENTSCHRIFT

(11) **EP 1 169 335 B1**
(45) Veröffentlichungstag und Bekanntmachung des Hinweises auf die Patenterteilung: **26.11.2003**
(21) Anmeldenummer: 00926783.2
(22) Anmeldetag: 31.03.2000
(51) Int. Cl.: C07J 9/00, C11B 13/00, C11B 13/02

(54) **VERFAHREN ZUR GEWINNUNG VON PHYTOSTERINEN DURCH KRISTALLISATION AUS VERMINDERTEN MENGEN METHANOLS**
METHOD FOR PRODUCING PHYTOSTEROLS FROM REDUCED QUANTITIES OF METHANOL BY CRYSTALLIZATION
PROCEDE DE PREPARATION DE PHYTOSTERINES PAR CRISTALLISATION A PARTIR DE QUANTITES REDUITES DE METHANOL

(30) Priorität: 09.04.1999 DE 19916034
(43) Veröffentlichungstag der Anmeldung: 09.01.2002
(73) Patentinhaber: Cognis Deutschland GmbH & Co. KG, 40589 Düsseldorf (DE)
(72) Erfinder: SICRE, Christophe, F-31800 Landorthe (FR); ARMENGAUD, René, F-31360 Boussens (FR); SCHWARZER, Jörg, D-40723 Hilden (DE); GUTSCHE, Bernhard, D-40724 Hilden (DE); MUSHOLT, Markus, D-48703 Stadtlohn (DE); JORDAN, Volkmar, D-48565 Steinfurt (DE)
(86) Internationale Anmeldenummer: EP0002849
(87) Internationale Veröffentlichungsnummer: WO00061603

(56) Entgegenhaltungen:
- WO-A-96/10033
- GB-A- 635 497
- US-A- 4 420 427
- US-A- 5 424 457
- US-A- 5 487 817
- DATABASE WPI Section Ch, Week 198617 Derwent Publications Ltd., London, GB; Class B01, AN 1986-109257 XP002146084 & JP 61 050996 A (DAICEL CHEM IND LTD), 13. März 1986 (1986-03-13)

## Beschreibung

### Gebiet der Erfindung

Die Erfindung befindet sich auf dem Gebiet der Lebensmittelzusatzstoffe und betrifft ein neues Verfahren zur vereinfachten Herstellung von Phytosterinen.

### Stand der Technik

Phytosterine und deren Ester weisen hypocholesterinämische Eigenschaften auf, d.h. diese Stoffe sind in der Lage, den Cholesteringehalt im Blut zu reduzieren. Sie werden daher als Nahrungsmittelzusatzstoffe beispielsweise zur Herstellung von Margarine, Fritierölen, Wurst, Speiseeis und dergleichen eingesetzt. Die Gewinnung von Sterinen und anderen unverseifbaren Bestandteilen, wie z.B. Tocopherolen, aus Destillaten, die bei der Entsäuerung von pflanzlichen Ölen anfallen, ist in der Patentliteratur bereits verschiedentlich beschrieben worden. Stellvertretend seien hier die Druckschriften **EP-A2 0610742** (Hoffmann-LaRoche), **GB-A1 2145079** (Nisshin Oil Mills Japan) und **EP-A1 0333472** (Palm Oil Research and Development Board) genannt.

Aus dem Europäischen Patent **EP-B1 0656894** (Henkel) ist ein Verfahren zur Herstellung von Sterinen bekannt, bei dem man einen Rückstand aus der Methylesterdestillation, der im wesentlichen aus Glyceriden, Sterinen, Sterinestern und Tocopherolen besteht, in Gegenwart alkalischer Katalysatoren mit Methanol umestert. Nach Neutralisation des Katalysators, destillativer Abtrennung des überschüssigen Methanols und gegebenenfalls Auswaschen des Katalysators erfolgt die Kristallisation der Sterine durch Absenken der Reaktionstemperatur von etwa 65 auf 20 °C. Die dabei anfallenden Kristalle werden anschließend mit Methanol und Wasser gewaschen. Die Ausbeute und die Farbqualität der Sterinen lässt jedoch zu wünschen übrig.

Demzufolge hat die Aufgabe der vorliegenden Erfindung darin bestanden, Phytosterine in hohen Ausbeuten und guter Farbqualität zur Verfügung zu stellen und bestehende Verfahren des Stands der Technik zu vereinfachen.

### Beschreibung der Erfindung

Gegenstand der Erfindung ist Verfahren zur Gewinnung von Phytosterinen aus Gemischen mit Fettsäuremethylestern und Methanol durch an sich bekannte Kristallisation, Filtration, Wäsche und Trocknung, welches sich dadurch auszeichnet, dass man das Methanol ― bezogen auf die Sterine ― in Mengen von 25 bis 75 Gew.-% einsetzt und die Rohsterine nach der Kristallisation mit Fettsäuremethylestern wäscht.

Überraschenderweise wurde gefunden, dass die Kristallisationstemperatur der Sterine signifikant durch den Methanolgehalt in der Reaktionsmischung beeinflusst wird. So erhöht sich die Schmelztemperatur einer Mischung mit einem Methanolgehalt von 30 Gew.-% gegenüber einer alkoholfreien Fraktion von 65 auf 78°C. Dies führt nicht nur zu einer Vereinfachung des Verfahrens und zu einer Verbesserung in der Energiebilanz, bei der anschließenden Aufarbeitung werden auch deutlich höhere Ausbeuten erzielt. Die Erfindung schließt dabei die Erkenntnis ein, dass der Anstieg der Kristallisationstemperatur nicht eine lineare Funktion des Methanolgehaltes ist, sondern bei Gehalten oberhalb von etwa 75 Gew.-% wieder ein rascher Abfall zu beobachten ist. Durch Waschen der Sterinkristalle mit Fettsäuremethylestern lässt sich zudem die Farbqualität der Produkte wesentlich verbessern.

### Umesterung

Die Herstellung einer sterinreichen Fraktion durch Umesterung von Rückständen aus der Entsäuerung von Pflanzenölen und nachfolgende Aufarbeitung kann in der aus der EP-B1 0656894 bekannten Weise erfolgen. Als Ausgangsstoffe eignen sich Destillationsrückstände, die beispielsweise als sogenannte Dämpferkondensate bei der Herstellung von Fettsäuremethylestern auf Basis von Rapsöl, Sojaöl oder insbesondere Sonnenblumenöl anfallen. Weiterhin geeignet ist Tallölpech, insbesondere Pech das aus Birkenrinde gewonnen wird. Soweit es die Herstellung der Sterinfraktionen betrifft, wird auf die oben genannte Druckschrift umfassend Bezug genommen. Das Verfahren eignet sich in besonderer Weise zur Herstellung von Sterinen auf Basis von Pflanzenölen, die nur einen geringen Anteil an α-Sitosterinen aufweisen. Als bevorzugtes Ausgangsmaterial dienen daher phytosterinreiche Fraktionen aus der Umesterung von Rapsöl ("Rapssterine") oder Sojaöl ("Sojasterine").

### Kristallisation

Die Kristallisation der Sterinfraktionen, die neben dem Alkohol überwiegend Methylester enthalten, erfolgt in an sich bekannter Weise, d.h. die heißen Mischungen (ca. 90 bis 100°C) werden in einem Kristallisator langsam bis auf ca. 10°C abgekühlt. Falls erforderlich kann im Gemisch enthaltener alkalischer Katalysator aus der Umesterung zuvor beispielsweise durch Zugabe von Zitronensäure neutralisiert werden. Im Sinne der Erfindung ist es erforderlich, entweder nur solche Gemische einzusetzen, welche schon herstellungsbedingt ein Gewichtsverhältnis Sterin : Methanol von 100 : 25 bis 100 : 75 aufweisen. Andernfalls muss Methanol zugesetzt bzw. abdestilliert werden. Die Kristallisation setzt unter diesen Bedingungen bei Temperaturen im Bereich von 75 bis 80°C ein. Es ist selbstverständlich auch möglich, anstelle der Umesterungsprodukte Roh-Sterine einzusetzen, mit Methanol und gegebenenfalls Methylester zu versetzen und in der beschriebenen Weise aufzukonzentrieren. Abschließend werden die Rohsterine mit Methylesterfraktionen gewaschen; hierbei gehen zwar geringe Produktmengen verloren, es wird jedoch eine nachhaltige Farbverbesserung erreicht. Die anfallenden Phytosterine werden dann in an sich bekannter Weise abgetrennt und aufgereinigt, d.h. abfiltriert, esterfrei gewaschen und bis zur Gewichtskonstanz getrocknet.

### Beispiele

### Vergleichsbeispiel V1

Als Ausgangsmaterial diente eine Rapsmethylesterfraktion, welche bezogen auf den Gehalt an freien und gebundenen Sterinen zusätzlich noch 100 Gew.-% Methanol enthielt. Die Mischung wurde von ca. 100°C kontinuierlich bis auf 10°C abgekühlt, wobei sich die ersten Kristalle schon bei 68°C abzuscheiden begannen. Nach Abschluss der Kristallisation wurden die Kristalle abfiltriert, mit Methanol methylesterfrei gewaschen und bis zur Gewichtskonstanz getrocknet. Die Ausbeute betrug - bezogen auf den Steringehalt im Umesterungsprodukt - 78 Gew.-%.

### Vergleichsbeispiel V2

Beispiel V1 wurde unter Einsatz einer Mischung wiederholt, welche bezogen auf die Sterinmenge 200 Gew.-% Methanol enthielt. Die Kristallisation setzte hier erst bei 63°C ein, die Ausbeute betrug 72 Gew.-%. Die Produkte besitzen in 10 Gew.-%iger ethanolischer Lösung eine Hazen-Farbzahl von 798 bzw. eine Gardner-Farbzahl von 4,4.

### Vergleichsbeispiel V3

Beispiel V1 wurde unter Einsatz einer Mischung wiederholt, welche bezogen auf die Sterinmenge 300 Gew.-% Methanol enthielt. Die Kristallisation setzte hier erst bei 56°C ein, die Ausbeute betrug 68 Gew.-%.

### Beispiel 1

Beispiel V1 wurde unter Einsatz einer Mischung wiederholt, welche bezogen auf die Sterinmenge 30 Gew.-% Methanol enthielt. Die Kristallisation setzte schon bei 78°C ein, die Ausbeute betrug 92 Gew.-%

### Beispiel 2

100 g einer rohen Sojasterinmischung (Steringehalt : 83 Gew.-%) wurde bei 90°C in 186 g Kokosfettsäuremethylester gelöst und mit einer solchen Menge Methanol versetzt, dass sich Gewichtsverhältnis von Sterin zu Methanol von 2 : 1 ergab. Nach Absenken der Temperatur wurden die ersten Sterinkristalle bereits bei 74°C abgeschieden. Nach Beendigung der Kristallisation wurden die Kristalle abfiltriert, mit Methanol methylesterfrei gewaschen und getrocknet. Die resultierende Fraktion zeigte eine Reinheit von 93,7 Gew.-%.

## Patentansprüche

1. Verfahren zur Gewinnung von Phytosterinen aus Gemischen mit Fettsäuremethylestern und Methanol durch an sich bekannte Kristallisation, Filtration, Wäsche und Trocknung, **dadurch gekennzeichnet, dass** man das Methanol ― bezogen auf die Sterine ― in Mengen von 25 bis 75 Gew.-% einsetzt und die Rohsterine nach der Kristallisation mit Fettsäuremethylestern wäscht.

2. Verfahren nach Anspruch 1, **dadurch gekennzeichnet, dass** man Raps- und/oder Sojasterine einsetzt.

## Claims

1. A process for the recovery of phytosterols from mixtures with fatty acid esters and methanol by crystallization known per se, filtration, washing and drying, **characterized in that** methanol is used in quantities of 25 to 75% by weight, based on the sterols, and the crude sterols are washed with fatty acid esters, after crystallation.

2. A process as claimed in claim 1, **characterized in that** rapessed and/or soya sterols are used.

## Revendications

1. Procédé de production de phytostérols à partir de mélanges avec des esters méthyliques d'acide gras et du méthanol par cristallisation, filtration, lavage et séchage connus en soi,
**caractérisé en ce qu'**
on met en oeuvre le méthanol ― rapporté aux stérols ― en quantités allant de 25 à 75 % en poids et on lave les stérols bruts après la cristallisation avec des esters de méthyle d'acide gras.

2. Procédé selon la revendication 1,
**caractérisé en ce qu'**
on utilise des stérols de colza et/ou de soja.
